(19) Europäisches Patentamt
European Patent Office
Office européen des brevets

(11) Veröffentlichungsnummer : **0 495 750 A2**

(12) # EUROPÄISCHE PATENTANMELDUNG

(21) Anmeldenummer : **92810004.9**

(22) Anmeldetag : **06.01.92**

(51) Int. Cl.[5] : **C07D 211/40,** C07D 211/42, C07D 211/44, A61K 31/445, C07D 211/22, C07D 241/04, C07D 265/30, C07D 279/12

(30) Priorität : **14.01.91 CH 86/91**

(43) Veröffentlichungstag der Anmeldung : **22.07.92 Patentblatt 92/30**

(84) Benannte Vertragsstaaten : **AT BE CH DE DK ES FR GB GR IT LI LU NL PT SE**

(71) Anmelder : **CIBA-GEIGY AG**
**Klybeckstrasse 141**
**CH-4002 Basel (CH)**

(72) Erfinder : **Frei, Jörg, Dr.**
**Buechring 36**
**CH-4434 Hölstein (CH)**
Erfinder : **Stanek, Jaroslav, Dr.**
**Hangstrasse 9**
**CH-4144 Arlesheim (CH)**

(54) **Heterozyklische Hydroxylamine.**

(57) Verbindungen der Formel

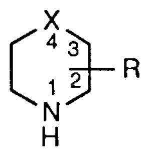

(I),

worin X für Methylen, Imino, Sauerstoff oder Schwefel steht und R einen Rest

$$-(CH_2)_n-O-NH_2$$

bezeichnet, worin n 0, 1 oder 2 bedeutet, mit der Massgabe, dass n nicht 0 bedeutet, wenn R in Position 2 gebunden ist, und Salze davon haben eine starke spezifische Hemmwirkung auf das Enzym Ornithindecarboxylase. Die Verbindungen der Formel I werden nach an sich bekannten Verfahren hergestellt.

EP 0 495 750 A2

Die Erfindung betrifft Verbindungen der Formel

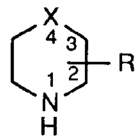

(I),

worin X für Methylen, Imino, Sauerstoff oder Schwefel steht und R einen Rest

$$-(CH_2)_n-O-NH_2$$

bezeichnet, worin n 0, 1 oder 2 bedeutet, mit der Massgabe, dass n von 0 verschieden ist, wenn R in Position 2 gebunden ist, und ihre Salze, Verfahren zur Herstellung dieser Verbindungen, pharmazeutische Präparate, die diese Verbindungen enthalten, die Verwendung dieser Verbindungen zur therapeutischen Behandlung des menschlichen oder tierischen Körpers und zur Herstellung pharmazeutischer Präparate.

Im Rahmen der vorliegenden allgemeinen Beschreibung erfolgt die Bezifferung der Ringatome in der Weise, dass ausgehend vom Stickstoff dieser mit 1 und X mit 4 beziffert wird, während in den Beispielen die Bezifferung nach der IUPAC-Nomenklatur erfolgt.

Salze von erfindungsgemässen Verbindungen sind in erster Linie pharmazeutisch annehmbare Säureadditionssalze, z.B. mit anorganischen Säuren, wie Salzsäure, Schwefelsäure oder Phosphorsäure, oder mit geeigneten organischen Carbon- oder Sulfonsäuren, z.B. Essigsäure, Octansäure, Bernsteinsäure, Adipinsäure, Fumarsäure, Maleinsäure, Hydroxymaleinsäure, Propionsäure, Milchsäure, Aepfelsäure, Zitronensäure, Salicylsäure, p-Aminosalicylsäure, Ascorbinsäure, Oxalsäure, Benzolsulfonsäure, 1,5-Naphthalindisulfonsäure, Methansulfonsäure oder 1,2-Ethandisulfonsäure, oder N-Cyclohexylsulfaminsäure, oder z.B. mit Aminosäuren, wie Glutaminsäure oder Asparaginsäure. Es können Mono- bis Trisalze gebildet werden, je nach Anzahl der vorhandenen basischen Gruppen.

Zur Isolierung oder Reinigung können auch pharmazeutisch ungeeignete Salze, z.B. Pikrate oder Perchlorate, Verwendung finden. Zur therapeutischen Anwendung gelangen nur die pharmazeutisch annehmbaren Salze, die deshalb bevorzugt sind.

Die Verbindungen der vorliegenden Erfindung, die über ein asymmetrisches Kohlenstoffatom verfügen, können in Form von reinen Enantiomeren oder als Enantiomerengemische (Racemate) vorliegen.

Die erfindungsgemässen Verbindungen weisen wertvolle, insbesondere pharmakologisch verwendbare, Eigenschaften auf. Insbesondere haben sie eine starke, spezifische Hemmwirkung auf das Enzym Ornithindecarboxylase (ODC). ODC spielt als ein Schlüsselenzym eine wichtige Rolle bei der Polyaminbiosynthese, die in praktisch allen Zellen von Säugetieren, einschliesslich Menschen, abläuft. Durch ODC wird die Polyaminkonzentration in der Zelle reguliert. Eine Hemmung des Enzyms ODC hat eine Verringerung der Polyaminkonzentration zur Folge. Da eine Verringerung der Polyaminkonzentration eine Hemmung des Zellwachstums bewirkt, ist es möglich, durch Verabreichung von ODC-hemmenden Substanzen das Wachstum sowohl von eukaryotischen als auch prokaryotischen, inbesondere von rasch oder unkontrollierbar wachsenden, Zellen zu hemmen und sogar Zellen abzutöten oder das Einsetzen der Zelldifferenzierung zu hemmen.

Die Hemmung des Enzyms ODC kann z.B. mit der Methode von J.E. Seely und A.E. Pegg, Ornithin Decarboxylase (mouse kidney), Seiten 158-161, in H. Tabor und C. White-Tabor (Hrsg.): Methods in Enzymology, Vol. 94: Polyamines, Academic Press, New York 1983, nachgewiesen werden. Wenn man in diesem Test ODC aus Rattenleber verwendet (Isolierung: Hayashi, S.-I. and Kameji, T., selber Band, Seiten 154- 158), werden $IC_{50}$-Werte im mikromolaren Bereich, z. B. zwischen 0,2 und 2 $\mu$M, erhalten. $IC_{50}$ ist die Konzentration des Inhibitors, bei der die ODC-Aktivität 50 % der Kontrolle ohne Inhibitor beträgt.

Als ODC-Hemmer besitzen die Verbindungen der Formel I antiproliferative Eigenschaften, die sich z.B. durch Nachweis der Hemmwirkung auf das Wachstum von menschlichen T24 Blasenkarzinomzellen demonstrieren lassen. Der Nachweis erfolgt, indem man diese Zellen in "Eagle's minimal essential medium ", dem 5 % (V/V) fötales Kälberserum zugesetzt werden, in einem befeuchteten Inkubator bei 37°C und 5 -Volumenprozent $CO_2$ in der Luft inkubiert. Die Karzinomzellen (1000-1500) werden in 96-Loch-Mikrotiterplatten überimpft und über Nacht unter den genannten Bedingungen inkubiert. Die Testsubstanz wird in seriellen Verdünnungen am Tag 1 hinzugefügt. Die Platten werden unter den genannten Bedingungen 5 Tage lang inkubiert. Während dieser Zeitspanne durchlaufen die Kontrollkulturen mindestens 4 Zellteilungen. Nach der Inkubation werden die Zellen mit 3,3%iger (Gewicht/Volumen) wässriger Glutaraldehydlösung fixiert, mit Wasser gewaschen und mit 0,05%iger (G/V) wässriger Methylenblaulösung gefärbt. Nach dem Waschen wird der Farbstoff mit 3%iger (G/V) wässriger Salzsäure eluiert. Danach wird die optische Dichte (OD) pro Loch, welche der Zellanzahl direkt proportinal ist, mit einem Photometer (Titertek multiskan) bei 665 nm gemessen. Die $IC_{50}$-Werte werden mit einem Computersystem unter Verwendung der Formel

$$\frac{OD_{(Test)} - OD_{665}(Anfang)}{OD_{665}(Kontrolle) - OD_{665}(Anfang)} \times 100$$

errechnet. Die $IC_{50}$-Werte sind als diejenige Wirkstoffkonzentration definiert, bei der die Anzahl der Zellen pro Loch am Ende der Inkubationszeit nur 50 % der Zellanzahl in den Kontrollkulturen beträgt.

Die Verbindungen der Formel I sind z.B. geeignet zur Behandlung von Krankheitszuständen, die auf eine Hemmung der Ornithindecarboxylase ansprechen, z. B. benigner und maligner Tumoren. Sie können Tumorregressionen bewirken und ferner die Verbreitung von Tumorzellen sowie das Wachstum von Mikrometastasen verhindern. Des weiteren können sie z.B. zur Behandlung von Protozoainfektionen, wie etwa Trypanosomiasis, Malaria oder durch Pneumocystis carinii verursachte Lungenentzündung, dienen.

Als selektive ODC-Hemmer können die Verbindungen der Formel I allein oder auch in Kombination mit anderen pharmakologisch wirksamen Substanzen angewendet werden. Zu denken ist z. B. an eine Kombination mit (a) Inhibitoren anderer Enzyme der Polyaminbiosynthese, z. B. S-Adenosylmethionindecarboxylasehemmern, (b) Inhibitoren der Proteikinase C, (c) Inhibitoren der Tyrosinproteinkinase, (d) Cytokinen, (e) negativen Wachstumsregulatoren, (f) Aromatasehemmern, (g) Antiöstrogenen oder (h) klassischen zytostatischen Wirkstoffen.

Bevorzugt betrifft die Erfindung Verbindungen der Formel I, in denen X für Methylen, Imino oder Sauerstoff steht, R in Position 2 oder 3 gebunden ist und n 1 oder 2 ist und Verbindungen der Formel I, in denen X Methylen bedeutet, R in Position 4 gebunden ist, und n 0 oder 1 ist, und Salze davon.

Hervorzuheben sind Verbindungen der Formel I, in denen X für Methylen oder Sauerstoff steht, R in Position 2 oder 3 gebunden ist und n 1 oder 2 ist und Verbindungen der Formel I, in denen X Methylen bedeutet, R in Position 4 gebunden ist, und n 0 oder 1 ist, und Salze davon.

Besonders bevorzugt sind Verbindungen der Formel I, in denen X für Methylen oder Sauerstoff steht, R in Position 3 gebunden ist und n 1 ist oder worin X für Methylen steht, R in Position 4 gebunden ist, und n 0 ist, und Salze davon.

Die Erfindung betrifft vor allem die in den Beispielen beschriebenen spezifischen Verbindungen und pharmazeutisch annehmbare Salze davon.

Die neuen Verbindungen der Formel I können in an sich bekannter Weise hergestellt werden, indem man z.B.

(a) aus einer Verbindung der Formel I, worin mindestens eine Aminogruppe geschützt ist, z.B. einer Verbindung der Formel

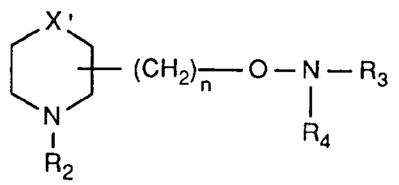
(II)

oder einem Salz davon, worin X' für Methylen, Sauerstoff, Schwefel oder N-$R_1$ steht, n 0, 1 oder 2 bedeutet, mit der Massgabe, dass n von 0 verschieden ist, wenn der Substituent $-(CH_2)_n-O-NR_3R_4$ in Position 2 gebunden ist, und $R_1$, $R_2$, $R_3$ und $R_4$ unabhängig voneinander für eine Aminoschutzgruppe oder Wasserstoff stehen, mit der Massgabe, dass mindestens eine der Gruppen $R_1$, $R_2$, $R_3$ und $R_4$ eine Aminoschutzgruppe bedeutet, oder worin $R_1$ und $R_2$ unabhängig voneinander für eine Aminoschutzgruppe oder Wasserstoff stehen und $R_3$ zusammen mit $R_4$ eine bivalente Aminoschutzgruppe bildet, die Aminoschutzgruppe(n) abspaltet, oder

(b) zur Herstellung von Verbindungen der Formel I oder eines Salzes davon, worin X Imino oder Methylen bedeutet, eine Verbindung der Formel

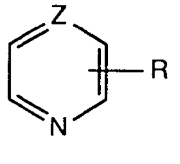
(III)

oder ein Salz davon, worin Z für Stickstoff oder die Methingruppe CH steht, und R die unter Formel I angegebene Bedeutung hat, reduziert, oder

(c) zur Herstellung von Verbindungen der Formel I oder eines Salzes davon, worin X Imino, Sauerstoff oder Schwefel bedeutet, R in Position 2 oder 3 gebunden ist und n 1 oder 2 ist, eine Verbindung der Formel

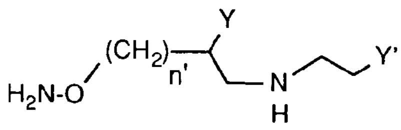

(IV),

worin n′ für 1 oder 2 steht, und einer der Reste Y und Y′ Hydroxyl, Amino oder Sulfhydryl darstellt, und der andere eine nucleofuge Abgangsgruppe bedeutet, oder ein Salz davon cyclisiert, oder
(d) eine Verbindung der Formel

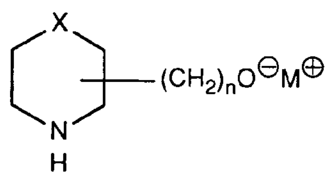

(V)

oder ein Salz davon, worin X und n die unter Formel I angegebenen Bedeutungen haben, mit der Massgabe, dass n von 0 verschieden ist, wenn der Substituent $-(CH_2)_nO^{\ominus}M^{\oplus}$ in Position 2 gebunden ist, und $M^{\oplus}$ für ein Metallkation steht, mit einer Verbindung der Formel $H_2N\text{-}A'$ oder einem Salz davon, worin A′ eine nucleofuge Abgangsgruppe ist, umsetzt, und
gewünschtenfalls eine erhältliche Verbindung der Formel I in eine andere Verbindung der Formel I überführt, ein erhältliches Enantiomerengemisch in die Enantiomeren aufspaltet und/oder eine erhältliche freie Verbindung der Formel I in ein Salz überführt oder ein erhältliches Salz in die freie Verbindung der Formel I oder in ein anderes Salz umwandelt.

Salze von Ausgangsmaterialien, die mindestens ein basisches Zentrum aufweisen, beispielsweise Verbindungen der Formel III, sind entsprechende Säureadditionssalze.

Das nachstehend verwendete Präfix "Nieder" bezeichnet im Rahmen der vorliegenden Anmeldung einen Rest mit 1 bis 7 und insbesondere mit 1 bis 4 Kohlenstoffatomen. Niederalkyl ist z.B. Methyl, Ethyl, Propyl, Butyl, Pentyl, Hexyl, Heptyl, wobei ein Alkylrest mit 3 bis 7 Kohlenstoffatomen geradkettig oder verzweigt vorliegen kann.

Verfahren (a):

Bevorzugte monovalente Aminoschutzgruppen $R_1$, $R_2$, $R_3$ und $R_4$ sind Acylgruppen, beispielsweise Niederalkanoyl, wie Formyl, Acetyl oder Propionyl, Halogenniederalkanoyl, wie 2-Halogenacetyl, insbesondere 2-Chlor-, 2-Brom-, 2-Jod-, 2,2,2-Trifluor oder 2,2,2-Trichloracetyl, gegebenenfalls, z.B. durch Halogen, Niederalkoxy, Niederalkoxycarbonyl oder Nitro substituiertes Benzoyl, z.B. Benzoyl, 4-Chlorbenzoyl, 4-Methoxybenzoyl, 2-Methoxycarbonyl-benzoyl oder 4-Nitrobenzoyl, Arylmethoxycarbonyl mit einem oder zwei Arylresten, die vorzugsweise gegebenenfalls z.B. durch Niederalkyl, insbesondere tert.-Niederalkyl, wie tert.-Butyl, Niederalkoxy, wie Methoxy, Hydroxy, Halogen, z.B. Chlor und/oder Nitro, mono- oder polysubstituiertes Phenyl darstellen, wie gegebenenfalls substituiertes Benzyloxycarbonyl, z.B. 4-Nitrobenzyloxycarbonyl, oder substituiertes Diphenylmethoxycarbonyl, z.B. Di-(4-methoxyphenyl)-methoxycarbonyl, 2-Halogenniederalkoxycarbonyl, z.B. 2,2,2-Trichlorethoxycarbonyl, 2-Brommethoxycarbonyl oder 2-Jodethoxycarbonyl, oder ein in 1-Stellung des Niederalkylrestes verzweigtes oder in 1- oder 2-Stellung geeignet substituiertes Niederalkoxycarbonyl, insbesondere tert.-Niederalkoxycarbonyl, z.B. tert.-Butoxycarbonyl, oder Arylmethylgruppen, wie Mono-, Di- oder insbesondere Triarylmethyl, wobei die Arylreste insbesondere gegebenenfalls substituierte Phenylreste darstellen, z.B. Benzyl, Diphenylmethyl oder Triphenylmethyl (Trityl).

Besonders bevorzugte monovalente Aminoschutzgruppen $R_1$, $R_2$, $R_3$ und $R_4$ sind Acylreste von Kohlensäurehalbestern, insbesondere tert.-Butoxycarbonyl, gegebenenfalls, z.B. wie oben angegeben, substituiertes Benzyloxycarbonyl, z.B. 4-Nitro-benzyloxycarbonyl, Diphenylmethoxycarbonyl, oder 2-Halogen-niederalkoxycarbonyl, wie 2,2,2-Trichlorethoxycarbonyl, ferner Trityl, Formyl oder 2-Methoxycarbonyl-benzoyl. Bevorzugte bivalente Aminoschutzgruppen, gebildet aus den Resten $R_3$ und $R_4$, sind mono- oder disubstituierte Methylidengruppen, wie 1-Niederalkoxy (beispielsweise Methoxy oder Ethoxy)-niederalkyliden (beispielsweise Ethyliden oder 1-n-Butyliden), z.B. $=C(CH_3)(OC_2H_5)$, ferner z.B. $=C(CH_3)_2$ oder =CH-Phenyl, und insbesondere Bisacylreste, z.B. der Phthalylrest, welcher zusammen mit dem zu schützenden Stickstoffatom ein 1H-isoindol-1,3(2H)-dion (Phthalimidogruppe) bildet.

Aminoschutzgruppen, ihre Einführung und Abspaltung sind an sich bekannt und z.B. in J.F.W. McOmie, "Protective Groups in Organic Chemistry", Plenum Press, London, New York, 1973, und T.W. Greene, "Protective Groups in Organic Synthesis", Wiley, New York, 1984 beschrieben.

Die Abspaltung der Aminoschutzgruppe(n) erfolgt, gegebenenfalls stufenweise oder gleichzeitig, je nach Art der Schutzgruppe(n) in an sich bekannter Weise, z.B. mittels Reduktion oder Solvolyse, insbesondere Hydrolyse, bevorzugt in saurem Medium, Alkoholyse, Acidolyse oder Hydrazinolyse. Die tert.-Butyloxycarbonylgruppe oder den Tritylrest kann man z.B. durch Behandeln mit einer Säure, wie Mineralsäure, z.B. Chlorwasserstoffsäure, gegebenenfalls in Gegenwart von Lösungsmitteln, insbesondere Methanol oder Tetrahydrofuran, oder einer organischen Säure, z.B. Ameisen-, Essig- oder Trifluoressigsäure, gegebenenfalls in Gegenwart von Wasser oder eines organischen Lösungsmittels, z.B. Methylenchlorid, freisetzen. Die Abspaltung der gegebenenfalls substituierten Benzyloxycarbonylgruppe gelingt z.B. reduktiv durch Hydrogenolyse, d.h. durch Behandeln mit Wasserstoff in Gegenwart eines geeigneten Katalysators, z.B. Palladium, oder mittels Natrium in flüssigem Ammoniak, oder durch Acidolyse, insbesondere mittels Bromwasserstoff/Eisessig. 2-Halogenniederalkoxycarbonyl kann z.B. durch Behandeln mit einem geeigneten Reduktionsmittel, wie Zink, in Gegenwart eines organischen Lösungsmittels, wie Methanol oder wässriger Essigsäure, abgespalten werden. Die Abspaltung der Phthalylgruppe kann z.B. mittels Hydrazinhydrat geschehen oder mittels einer Säure, z.B. einer Mineralsäure, wie Chlorwasserstoffsäure, gegebenenfalls in Gegenwart von organischen Lösungsmitteln, z.B. Methanol.

Die Ausgangsverbindungen der Formel II sind bekannt oder werden nach an sich bekannten Verfahren hergestellt [vgl. z.B. Houben-Weyl, Methoden der Organischen Chemie, Bd X/1 (1971) und Bd. E 16a (1990)].

Die Ausgangsverbindungen der Formel II werden z.B. hergestellt, indem man eine Verbindung der Formel

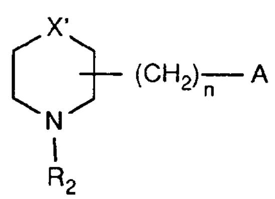

(VI)

worin X', $R_2$ und n die unter Formel II angegebene Bedeutung haben und A für Hydroxy oder eine nucleofuge Abgangsgruppe steht, mit gegebenenfalls N-geschütztem Hydroxylamin umsetzt.

Bedeutet in einer Verbindung der Formel VI A Hydroxy, so kann man z.B. eine intermolekulare Dehydratisierungsreaktion durchführen. Insbesondere kommt dafür eine Variante der Mitsunobu-Reaktion [Synthesis (1976), 682] in Frage, bei der die Verbindung der Formel VI mit einem N-geschützten Hydroxylamin-Derivat, wie z.B. N-Hydroxyphthalimid, N-Hydroxy-5-norbomen-2,3-dicarbonsäureimid oder Acethydroxamsäureethylester, und z.B. Triphenylphosphin und N,N'-Azodicarbonsäurediethylester umgesetzt wird.

In den Verbindungen der Formel VI ist die nucleofuge Abgangsgruppe A z.B. aliphatisch oder aromatisch - substituiertes Sulfonyloxy, z.B. Methansulfonyloxy oder p-Toluolsulfonyloxy (Tosyloxy), ferner aber auch z.B. Halogen, insbesondere Chlor, Brom oder Jod. Entsprechende Verbindungen der Formel VI werden in einer einfachen nucleophilen Substitution mit N-geschützten Hydroxylamin-Derivaten, z.B. den oben genannten, umgesetzt.

Verbindungen der Formel II können auch dadurch hergestellt werden, dass man eines der Verfahren (b), (c) oder (d) mit geschützter (geschützten) Aminogruppe(n) durchführt. Ferner können Verbindungen der Formel II auch aus Verbindungen der Formel I - etwa zu Reinigungszwecken - hergestellt werden.

Verfahren (b):

Verbindungen der Formel III werden durch Reduktion, z.B. mit einem Erdalkali- oder Alkalimetall, z.B. Natrium, in einem Niederalkanol, wie Ethanol, elektrolytische Reduktion, durch Reduktion mit komplexen Metallhydriden, z.B. Natrium- oder Lithiumaluminiumhydrid, Natrium- oder Lithiumborhydrid, oder insbesondere mit Wasserstoff in Gegenwart eines Uebergangsmetall-Katalysators, in die entsprechenden Hexahydroderivate der Formel I überführt [vgl. M. Hudlicky, Reductions in Organic Chemistry, Ellis Horwood Limited (1984)]. Die katalytische Hydrierung wird beispielsweise mit Palladium, Platinoxid oder Rhodium gegebenenfalls unter Zusatz von Kohle, bevorzugt in saurem Milieu, z.B. in Eisessig, oder mit Rutheniumdioxid, Raney-Nickel oder Kupferchromit durchgeführt. Verbindungen der Formel III sind bekannt oder werden nach an sich bekannten Verfahren hergestellt.

Verfahren (c):

Eine nucleofuge Abgangsgruppe Y oder Y′ ist z.B. Hydroxy oder eine reaktionsfähig veresterte Hydroxygruppe, wie mit einer starken anorganischen Säure oder organischen Sulfonsäure verestertes Hydroxy, beispielsweise Halogen, wie Chlor, Brom oder Iod, Sulfonyloxy, wie Hydroxysulfonyloxy, Halogensulfonyloxy, z.B. Fluorsulfonyloxy, gegebenenfalls, z.B. durch Halogen, substituiertes Niederalkansulfonyloxy, z.B. Methan- oder Trifluormethansulfonyloxy, Cycloalkansulfonyloxy, z.B. Cyclohexansulfonyloxy, oder gegebenenfalls, z.B. durch Niederalkyl oder Halogen, substituiertes Benzolsulfonyloxy, z.B. p-Brombenzol- oder p-Toluolsulfonyloxy.

Vorzugsweise ist Y Hydroxy, Amino oder Sulfhydryl und Y′ eine nucleofuge Abgangsgruppe, z.B. eine der vorstehend genannten.

Verbindungen der Formel IV, worin einer der Reste Y oder Y′ Hydroxyl, Amino oder Sulfhydryl darstellt, und der andere eine nucleofuge Abgangsgruppe, z.B. eine der oben genannten, können z.B. in Gegenwart eines Kondensationsmittels, wie einer geeigneten Base, in Verbindungen der Formel I umgewandelt werden. Als Base kommen beispielsweise anorganische Basen, wie Alkalimetallhydroxide, z.B. Natronlauge, oder insbesondere organische Basen, z.B. Niederalkylamine, wie Di- oder Triethylamin, basische Heterozyklen, beispielsweise vom Pyridintyp, z.B. Pyridin, oder Alkalimetallalkoxide, z.B. Kalium-tert.-butylat, in Frage.

In einer besonders bevorzugten Ausführungsform wird beispielsweise eine Verbindung der Formel IV, worin Y Hydroxyl, Amino oder Sulfhydryl ist und Y′ Hydroxy bedeutet, mit aliphatisch - oder aromatisch - substituiertem Sulfonylchlorid, z.B. Methansulfonylchlorid oder Tosylchlorid (p-Toluolsulfochlorid) in Gegenwart einer anorganischen oder organischen Base, z.B. Pyridin, umgesetzt, wobei unter den Reaktionsbedingungen in situ eine Weiterreaktion zu dem gewünschten Endprodukt der Formel I bzw. einem Salz davon eintritt.

Insbesondere bei der zuletzt genannten Umsetzung kann es erforderlich sein, die Aminogruppen in den Verbindungen der Formel IV vor der Durchführung der Reaktion durch Aminoschutzgruppen, z.B. die in Verfahren (a) genannten, zu schützen. Nach der Reaktion werden die Aminoschutzgruppen dann in an sich bekannter Weise wieder abgespalten.

Verbindungen der Formel IV, worin Y und Y′ beide Hydroxy sind, können z.B. in Gegenwart starker Säuren, z.B. Schwefelsäure, oder Fluorwasserstoffsäure, gegebenenfalls in Gegenwart von Schwefeltetrafluorid, oder in Gegenwart saurer Ionenaustauscher zu Verbindungen der Formel I, worin X Sauerstoff bedeutet, cyclisiert werden.

Ferner können Verbindungen der Formel IV, worin einer der Reste Y oder Y′ Hydroxyl oder Sulfhydryl darstellt und der andere Hydroxy, z.B. durch Umsetzung mit Fluorierungsmitteln, die geeignet sind, z.B. Hydroxy in Fluor zu überführen, beispielsweise Fluorwasserstoff, Schwefeltetrafluorid, insbesondere ein Gemisch aus Fluorwasserstoff und Schwefeltetrafluorid und ferner auch substituierte Aminoschwefeltrifluoride, wie Diethylaminoschwefeltrifluorid (DAST) oder Piperidinoschwefeltrifluorid, zu Morpholinoder Thiomorpholinderivaten der Formel I cyclisiert werden.

Die Ausgangsverbindungen der Formel IV sind bekannt oder werden nach an sich bekannten Verfahren hergestellt.

Die Ausgangsverbindungen der Formel IV, worin Y und/oder Y′ aliphatisch - oder aromatisch - substituiertes Sulfonyloxy bedeutet, werden bevorzugt in an sich bekannter Weise aus Verbindungen der Formel IV, worin Y und/oder Y′ für Hydroxy steht, hergestellt, z.B. durch Reaktion mit Niederalkan- oder Arensulfonylchloriden.

Ferner können Ausgangsverbindungen der Formel IV, worin Y oder Y′ Amino oder Sulfhydryl darstellt, in situ hergestellt werden, indem man z.B. eine Verbindung der Formel

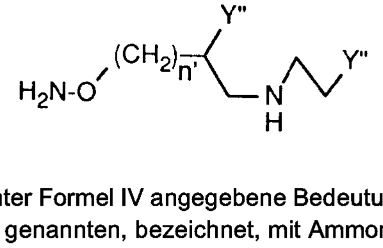

(IV')

worin n′ die unter Formel IV angegebene Bedeutung hat und Y″ jeweils eine nucleofuge Abgangsgruppe, z.B. eine der oben genannten, bezeichnet, mit Ammoniak oder Natriumsulfid umsetzt, wobei die primär gebildete Verbindung der Formel IV unter den gegebenen Reaktionsbedingungen gleich weiterreagiert.

Die Aminogruppen in den Verbindungen der Formel IV′ können gegebenenfalls vor Durchführung der Reaktion durch Aminoschutzgruppen, z.B. die in Verfahren (a) genannten, geschützt werden und auf einer beliebigen Verfahrensstufe in an sich bekannter Weise freigesetzt werden.

Die Ausgangsverbindungen der Formel IV, worin Y für Hydroxy oder Sulfhydryl und Y′ für Hydroxy steht, werden z.B. hergestellt, indem man eine Verbindung der Formel

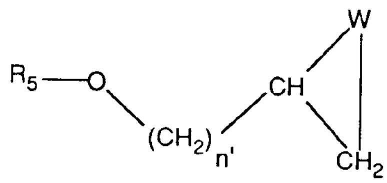

(VII)

worin n′ die unter Formel IV angegebene Bedeutung hat, W für Sauerstoff oder Schwefel steht und $R_5$ eine geschützte Aminogruppe bedeutet, z.B. eine der in Verfahren (a) genannten, oder für einen Rest

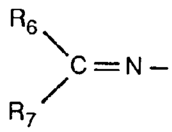

steht, worin z.B. $R_6$ Niederalkyl, Niederalkoxyniederalkyl, beispielsweise Methoxymethyl oder Ethoxymethyl, oder Phenyl bedeutet, $R_7$ für Wasserstoff, Niederalkyl oder Niederalkoxy, z.B. n-Propoxy, Isopropoxy, n-Butoxy oder tert.Butoxy, vorzugsweise Methoxy, und vor allem Ethoxy steht oder $R_5$ und $R_7$ zusammen $C_4$-$C_6$-Alkylen oder Benzo$C_4$-$C_6$-alkylen bedeuten, mit Ethanolamin umsetzt und anschliessend die geschützte Aminogruppe freisetzt, z.B. in der oben beschriebenen Weise oder die

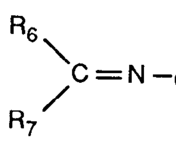 Gruppe hydrolysiert.

Unter einem Rest $R_5R_7C$=N-, worin $R_6$ und $R_7$ zusammen $C_4$-$C_6$-Alkylen bedeuten, ist Cycloalkylidenamino mit 5 bis 7 Ringkohlenstoffatomen, z.B. Cyclopentylidenamino oder Cyclohexylidenamino, zu verstehen.

Unter einem Rest $R_5R_7C$=N-, worin $R_6$ und $R_7$ zusammen Benzo-$C_4$-$C_6$-alkylen bedeuten, ist z.B. Cyclopentylidenamino oder Cyclohexylidenamino zu verstehen, welche jeweils einen annellierten Benzoring tragen.

Die Umsetzung von Verbindungen der Formel VII mit Ethanolamin wird ohne Lösungsmittel oder in Gegenwart eines Lösungsmittels, z.B. einem Niederalkanol oder Ether, wie Isopropanol oder Tetrahydrofuran, und gegebenenfalls unter erhöhtem Druck durchgeführt und erfolgt selektiv am terminalen C-Atom des Oxiranyl- bzw. des Thiiranylrestes.

Die Ausgangsverbindungen der Formel VII, worin W für Sauerstoff steht, werden z.B. dadurch erhalten, dass man ein Hydroxylamin bzw. Oxim der Formel $R_5$-OH mit z.B. Epichlorhydrin, Epibromhydrin oder 3-Tosyloxy-1,2-epoxypropan umsetzt. Ausgangsverbindungen der Formel VII, worin W für Schwefel steht, entstehen beispielsweise bei der Umsetzung eines Hydroxylamins bzw. Oxims der Formel $R_5$-OH mit Thioepichlorhydrin. Diese Reaktionen werden bevorzugt in Gegenwart einer Base, z.B. Natriumhydroxid, und ohne Lösungsmittel oder in Gegenwart eines Lösungsmittels, z.B. Aceton oder Acetonitril, durchgeführt.

Verfahren (d):

Ein Metallkation $M^{\oplus}$ steht beispielsweise für das Kation eines Alkalimetalls, z.B. Kalium, Natrium oder Lithium oder ein Kation 1/2 ($M_1^{2\oplus}$), wobei $M_1$ ein Erdalkcalimetall, wie Magnesium-, Calcium- oder Barium bezeichnet.

Eine nucleofuge Abgangsgruppe A′ ist z.B. eine reaktionsfähig veresterte Hydroxygruppe, beispielsweise eine der unter Verfahren (c) genannten.

In einer bevorzugten Ausführungsform werden z.B. Metallsalze der Formel V, worin $M^{\oplus}$ ein Natriumkation bedeutet, mit Halogenamin, beispielsweise Chloramin, oder dem Natriumsalz der Hydroxylamin-O-sulfonsäure zu einer Verbindung der Formel I umgesetzt.

Metallsalze der Formel V entstehen beispielsweise, wenn Metalle, insbesondere Erdalkalioder Allcalimetalle, unter Wasserstoffentwicklung auf entsprechende Alkohole einwirken oder durch die Umsetzung der Alkohole mit geeigneten Basen, wie einem Alkalimetallhydrid oder -amid.

Die Erfindung betrifft insbesondere die in den Beispielen beschriebenen Verbindungen und Verfahren.

Salze von Verbindungen der Formel (I) können in an sich bekannter Weise hergestellt werden, beispielsweise durch Behandeln mit einer Säure, wie einer anorganischen Säure, z. B. Salzsäure oder Schwefelsäure, einer organischen Carbonsäure, z. B. Adipinsäure, oder einer organischen Sulfonsäure, z. B. Benzolsulfonsäure oder einem geeigneten Anionenaustauscherreagenz, welches beispielsweise mit der entsprechenden Säure beladen ist. Salze können in üblicher Weise in die freien Verbindungen überführt werden, z.B. durch Behandeln mit einem geeigneten basischen Mittel, wie einer Hydroxybase in freier Lösung, z. B. einem Alkalimetallhydroxid, oder wie einem mit Hydroxid beladenen Anionenaustauscher, z. B. durch Chromatographie oder im Batch-Verfahren.

Auch ist die direkte Umwandlung eines Säureadditionssalzes aus einer der Verbindungen der Formel I und einer Säure mit einer anderen Säure in ein Säureadditionssalz aus der Verbindung der Formel I und der zweiten, neuen Säure möglich. Diese Umwandlung kann erfolgen a) durch Reaktion des Ausgangssäureadditionssalzes in freier Lösung in Gegenwart einer geeigneten Menge der neuen Säure, z. B. einem Überschuss, oder b) an einem mit dem Anion der neuen Säure beladenen Anionenaustauscher.

Für alle Reaktionen, die der Umwandlung von Säureadditionssalzen von Basen der Formel I in andere Säureadditionssalze oder in die freien Verbindungen oder von den freien Basen in die entsprechenden Säuren dienen, sind auch gelchromatographische Verfahren anwendbar.

Je nach Verfahrensweise bzw. Reaktionsbedingungen können die erfindungsgemässen Verbindungen mit salzbildenden basischen Eigenschaften in freier Form oder in Form von Salzen erhalten werden.

Die Verbindungen, einschliesslich ihrer Salze, können auch in Form ihrer Hydrate erhalten werden, oder ihre Kristalle können z.B. das zur Kristallisation verwendete Lösungsmittel einschliessen.

Erfindungsgemäss erhältliche Gemische von Enantiomeren können in an sich bekannter Weise in einzelne Enantiomere aufgetrennt werden, z.B. durch Bilden von Salzen mit optisch reinen salzbildenden Reagentien und Auftrennen des so erhältlichen Diastereomerengemisches, z.B. mittels fraktionierter Kristallisation.

Die oben angeführten Reaktionen können unter an sich bekannten Reaktionsbedingungen durchgeführt werden, in Ab- oder üblicherweise Anwesenheit von Lösungs- oder Verdünnungsmitteln, vorzugsweise solchen, die gegenüber den verwendeten Reagenzien inert sind und diese lösen, in Ab- oder Anwesenheit von Katalysatoren, Kondensationsmitteln oder neutralisierenden Agentien, je nach Art der Reaktion und/oder der Reaktionsteilnehmer bei erniedrigter, normaler oder erhöhter Temperatur, z.B. im Temperaturbereich von etwa -80°C bis etwa 190°C, vorzugsweise von etwa -20°C bis etwa 150°C, z.B. bei Raumtemperatur, - 20 bis 40 °C oder beim Siedepunkt des verwendeten Lösungsmittels, unter atmosphärischem Druck oder in einem geschlossenen Gefäss, gegebenenfalls unter Druck, und/oder in einer inerten Atmosphäre, z.B. unter einer Stickstoffatmosphäre.

Zu den Lösungsmitteln, aus denen die für die jeweilige Reaktion geeigneten ausgewählt werden können, zählen beispielsweise Wasser, Ether, wie aliphatische Ether, z. B. Diethylether, oder wie cyclische Ether, z. B. Tetrahydrofuran, flüssige aromatische Kohlenwasserstoffe, wie Benzol oder Toluol, Alkohole, wie Methanol, Ethanol oder 1- oder 2-Propanol, Nitrile, wie Acetonitril, Halogenkohlenwasserstoffe, wie Methylenchlorid, Säureamide, wie Dimethylformamid, Basen, wie heterocyclische Stickstoffbasen, z. B. Pyridin, oder Gemische dieser Lösungsmittel, z. B. wässrige Lösungen, soweit bei bei der Beschreibung der Verfahren nichts anderes angegeben ist.

In den Verfahren der vorliegenden Erfindung werden vorzugsweise solche Ausgangsstoffe eingesetzt, die zu den eingangs als besonders wertvoll beschriebenen Verbindungen führen.

Die Erfindung betrifft auch diejenigen Ausführungsformen des Verfahrens, bei denen man von einer auf einer beliebigen Verfahrensstufe als Zwischenprodukt erhältlichen Verbindung ausgeht und die fehlenden Verfahrensschritte durchführt oder man das Verfahren auf irgendeiner Stufe abbricht, oder bei denen ein Ausgangsstoff unter den Reaktionsbedingungen gebildet oder in Form eines Derivates, z.B. eines Salzes davon, verwendet wird.

Die vorliegende Erfindung betrifft ebenfalls pharmazeutische Präparate, die als Wirkstoff eine der pharmakologisch wirksamen Verbindungen der Formel I enthalten. Besonders bevorzugt sind Präparate zur enteralen, insbesondere oralen, sowie zur parenteralen Verabreichung. Die Präparate enthalten den Wirkstoff allein oder vorzugsweise zusammen mit einem pharmazeutisch anwendbaren Trägermaterial. Die Dosierung des Wirkstoffs hängt von der zu behandelnden Krankheit, sowie von Spezies, deren Alter, Gewicht und individuellem Zustand, sowie von der Applikationsweise ab.

Die pharmazeutischen Präparate enthalten von etwa 5 % bis etwa 95 % des Wirkstoffs, wobei einzeldosierte Applikationsformen vorzugsweise von etwa 20 % bis etwa 90 % und nicht-einzeldosierte Applikationsformen vorzugsweise etwa 5 % bis etwa 20 % Wirkstoff aufweisen. Dosiseinheitsformen, wie Dragées, Tabletten oder Kapseln, enthalten von etwa 0,05 g bis etwa 1,0 g des Wirkstoffs.

Die pharmazeutischen Präparate der vorliegenden Erfindung werden in an sich bekannter Weise, z.B. mittels konventioneller Misch-, Granulier-, Dragier-, Lösungs-, oder Lyophilisierungsverfahren hergestellt. So kann man pharmazeutische Zusammensetzungen zur oralen Anwendung erhalten, indem man den Wirkstoff mit einem oder mehreren festen Trägerstoffen kombiniert, ein erhaltenes Gemisch gegebenenfalls granuliert, und das Gemisch bzw. Granulat, wenn erwünscht, gegebenenfalls durch Zugabe von zusätzlichen Hilfsstoffen, zu Tabletten oder Dragées-Kernen verarbeitet.

Geeignete Trägerstoffe sind insbesondere Füllstoffe, wie Zucker, z.B. Lactose, Saccharose, Mannit oder Sorbit, Cellulosepräparate und/oder Calciumphosphate, z.B. Tricalciumphosphat oder Calciumhydrogenphosphat, ferner Bindemittel, wie Stärken, z.B Mais-, Weizen-, Reis- oder Kartoffelstärke, Methylcellulose, Hydroxypropylmethylcellulose, Natriumcarboxymethylcellulose und/oder Polyvinylpyrrolidon, und/oder, wenn erwünscht, Sprengmittel, wie die obgenannten Stärken, ferner Carboxymethylstärke, quervernetztes Polyvinylpyrrolidon, Alginsäure oder ein Salz davon, wie Natriumalginat. Zusätzliche Hilfsmittel sind in erster Linie Fliessregulier- und Schmiermittel, z.B. Kieselsäure, Talk, Stearinsäure oder Salze davon, wie Magnesium- oder Calciumstearat, und/oder Polyethylenglykol, oder Derivate davon.

Dragées-Kerne können mit geeigneten, gegebenenfalls Magensaft-resistenten Ueberzügen versehen werden, wobei man u.a. konzentrierte Zuckerlösungen, welche gegebenenfalls arabischen Gummi, Talk, Polyvinylpyrrolidon, Polyethylenglykol und/oder Titandioxid enthalten, Lacklösungen in geeigneten organischen Lösungsmitteln oder Lösungsmittelgemischen oder, zur Herstellung von Magensaft-resistenten Ueberzügen, Lösungen von geeigneten Cellulosepräparaten, wie Acetylcellulosephthalat oder Hydroxypropylmethylcellulosephthalat, verwendet. Den Tabletten oder Dragée-Ueberzügen können Farbstoffe oder Pigmente, z.B. zur Identifizierung oder zur Kennzeichnung verschiedener Wirkstoffdosen, beigefügt werden.

Oral anwendbare pharmazeutische Zusammensetzungen sind ebenfalls Steckkapseln aus Gelatine, sowie weiche, geschlossene Kapseln aus Gelatine und einem Weichmacher, wie Glycerin oder Sorbit. Die Steckkapseln können den Wirkstoff in Form eines Granulats, z.B. im Gemisch mit Füllstoffen, wie Maissträke, Bindemitteln und/oder Gleitmitteln, wie Talk oder Magnesiumstearat, und gegebenenfalls von Stabilisatoren, enthalten. In weichen Kapseln ist der Wirkstoff vorzugsweise in geeigneten flüssigen Hilfsstoffen, wie fetten Oelen, Paraffinöl oder flüssigen Polyethylenglykolen, gelöst oder suspendiert, wobei ebenfalls Stabilisatoren zugefügt sein können.

Weitere orale Applikationsformen sind z.B. in üblicher Weise bereitete Sirups, die den Wirkstoff z.B. in suspendierter Form und in einer Konzentration von ca. 5 % bis 20 %, vorzugsweise ca. 10 % oder in einer ähnlichen Konzentration, die z.B. beim Abmessen von 5 oder 10 ml eine geeignete Einzeldosis ergibt, enthalten. Ferner kommen z.B. auch pulverförmige oder flüssige Konzentrate zur Bereitung von Shakes, z.B. in Milch, in Betracht. Solche Konzentrate können auch in Einzeldosismengen abgepackt sein.

Als rektal anwendbare pharmazeutische Präparate kommen z.B. Suppositorien in Betracht, welche aus einer Kombination des Wirkstoffs mit einer Suppositoriengrundmasse bestehen. Als Suppositoriengrundmasse eignen sich z.B. natürliche oder synthetische Triglyceride, Paraffinkohlenwasserstoffe, Polyethylenglykole oder höhere Alkanole.

Zur parenteralen Verabreichung eignen sich in erster Linie wässrige Lösungen eines Wirkstoffes in wasserlöslicher Form, z.B. eines wasserlöslichen Salzes, oder wässrige Injektionssuspensionen, welche viskositätserhöhende Stoffe, z.B. Natriumcarboxymethylcellulose, Sorbit und/oder Dextran und gegebenenfalls Stabilisatoren enthalten. Dabei kann der Wirkstoff, gegebenenfalls zusammen mit Hilfsstoffen, auch in Form eines Lyophilisats vorliegen und vor der parenteralen Verabreichung durch Zugabe von geeigneten Lösungsmitteln in Lösung gebracht werden.

Lösungen, wie sie z.B. für die parenterale Verabreichung verwendet werden, können auch als Infusionslösungen angewandt werden.

Die Erfindung betrifft ebenfalls ein Verfahren zur Behandlung der oben genannten Krankheitszustände in Warmblütern, d.h. Mensch und Tier, insbesondere solchen Warmblütern, die einer derartigen Behandlung bedürfen. Die Verbindungen der vorliegenden Erfindung können prophylaktisch oder therapeutisch verabreicht werden, wobei man sie vorzugsweise in Form von pharmazeutischen Präparaten verwendet, beispielsweise in einer zur Hemmung der Ornithindecarboxylase geeigneten, prophylaktisch oder therapeutisch gegen eine der genannten Krankheiten, z. B. Tumoren oder Protozoainfektionen, wirksamen Dosis. Dabei wird bei einem Körpergewicht von etwa 70 kg eine tägliche Dosis von etwa 0,3 g bis etwa 15 g, vorzugsweise von etwa 0,5 g bis etwa 5 g einer Verbindung der vorliegenden Erfindung verabreicht. Die pharmazeutischen Präparate sind vorzugsweise solche, die geeignet sind, einem Warmblüter, z. B. Menschen, zur Therapie oder Prophylaxe einer der oben genannten Erkrankungen, die auf eine Hemmung der Ornithindecarboxylase anspricht, verabreicht zu werden und eine zur Hemmung dieses Enzyms wirksame Menge einer Verbindung der Formel I oder

ein Salz hiervon, gegebenenfalls zusammen mit wenigstens einem Trägermaterial, umfassen.

Die nachfolgenden Beispiele illustrieren die vorliegende Erfindung; Temperaturen werden in Grad Celsius angegeben. Folgende Abkürzugen werden verwendet: Essigester ≙ Essigsäureethylester, BOC ≙ tert.-Butyloxycarbonyl; Ether ≙ Diethylether, konz. ≙ konzentriert; $R_f$ ≙ Verhältnis von Laufstrecke zu Lösungsmittelfront bei Dünnschichtchromatographie; Smp. ≙ Schmelzpunkt; Zers. ≙ unter Zersetzung. Sofern nicht snders angegeben, nehmen Lösungsmittelverhältnisse Bezug auf Volumina (v/v).

Beispiel 1: 4-Aminooxy-piperidin-dihydrochlorid

Unter Rühren werden 16,1 g (0,04648 Mol) 2-(N-BOC-4-piperidyloxy)-1H-isoindol1,3(2H)-dion in 100 ml 6N Salzsäure suspendiert. Man erhitzt das Reaktionsgemisch 2 h bei 100°, wobei unter $CO_2$-Entwick1ung zunächst eine klare Lösung entsteht, aus der aber nach kurzer Zeit wieder ein kristalliner Niederschlag (Phthalsäure) ausfällt. Das auf 0°C abgekühlte Reaktionsgemisch wird filtriert, das Nutschgut mit Wasser gewaschen und das Filtrat im Vakuum eingedampft. Den Rückstand nimmt man in Ethanol auf und dampft im Vakuum zur Trockene ein. Nach erneuter Zugabe von Ethanol, Eindampfen im Vakuum und Umkristallisation des Rückstands aus Methanol/Ether erhält man die Titelverbindung, Smp. 182°C (Zers.).

Die Ausgangsverbindung wird wie folgt hergestellt:

a) 2-(N-BOC-4-piperidyloxy)-1H-isoindol-1,3(2H)-dion

Zu einer Lösung von 16 g (0,07947 Mol) N-BOC-4-hydroxy-piperidin (vgl. EP 0278621), 12,97 g (0,07947 Mol) N-Hydroxyphthalimid und 20,85 g (0,07947 Mol) Triphenylphosphin in 160 ml Tetrahydrofuran tropft man unter Rühren und Stickstoffatmosphäre bei 30-35°C 14,07 ml (0,08415 Mol) Azodicarbonsäure-diethylester (93 %). Das Reaktionsgemisch wird 2 h bei Raumtemperatur weitergerührt und dann im Vakuum eingedampft. Zur Abtrennung von 1,2-Hydrazindicarbonsäure-diethylester und Triphenylphosphinoxid löst man den öligen Rückstand in Essigester, kühlt auf 0°C ab, filtriert, dampft das Filtrat ein und wiederholt den gleichen Vorgang noch einmal, jedoch unter Verwendung von Ether. Der nach dem Eindampfen von Ether erhaltene Rückstand wird mittels Flashchromatographie an Kieselgel der Korngrösse 0,04 - 0,063 mm, unter Verwendung von Essigester-Hexan-Gemischen (1:2 bzw. 2:3), gereinigt. Nach Eindampfen der produkthaltigen Fraktionen und Umkristallisation des Rückstands aus Methylenchlorid/Hexan erhält man die Titelverbindung, Smp. 119-121°C.

2-(N-B0C-4-piperidyloxy)-1H-isoindol-1,3(2H)-dion kann auch ausgehend von 4-Hydroxy-piperidin auf folgendem Weg erhalten werden:

b) Ein Gemisch von 5,06 g (0,05 Mol) 4-Hydroxy-piperidin in 50 ml Toluol wird tropfenweise mit einer Lösung von 10,92 g (0,05 Mol) Di-tert.-butyl-dicarbonat in 30 ml Toluol versetzt und 4 Tage bei Raumtemperatur gerührt. Zu der so erhaltenen Lösung von N-BOC-4-hydroxy-piperidin in Toluol gibt man unter fortgesetztem Rühren und Stickstoffatmosphäre 8,16 g (0,05 Mol) N-Hydroxyphthalimid und 13,11 g (0,05 Mol) Triphenylphosphin. Bei 30-35°C wird dann eine Lösung von 8,85 ml (0,0529 Mol) Azodicarbonsäurediethylester (93 %) in 20 ml Toluol zugetropft und das Reaktionsgemisch noch 15 h bei Raumtemperatur gerührt. Man kühlt auf 5°C, filtriert den ausgefallenen Hydrazindicarbonsäure-diethylester ab, dampft das Filtrat im Vakuum ein und reinigt den Rückstand mittels Flashchromatographie an Kieselgel unter Verwendung von EssigesterHexan-Gemischen (1:3 bzw. 1:2). Nach Eindampfen der produkthaltigen Fraktionen erhält man die Titelverbindung als kristallinen Rückstand, Smp. 116- 118°C.

c) 4-Aminooxy-piperidin-dihydrochlorid kann auch wie folgt erhalten werden:

Ein Gemisch von 1,414 g (0,005 Mol) 2-(4-Piperidyloxy)-1H-isoindol-1,3(2H)dion-hydrochlorid und 10 ml 6N HCl wird 2 h bei 100° erhitzt und anschliessend analog Beispiel 1 aufgearbeitet, wobei man die Titelverbindung erhält, Smp. 182°C (Zers.).

Die Ausgangsverbindung wird wie folgt hergestellt:

d) 2-(4-Piperidyloxy)-1H-isoindol-1,3(2H)-dion-hydrochlorid

In eine Lösung von 3,46 g (0,01 Mol)2-(N-BOC-4-piperidyloxy)-1H-isoindol-1,3(2H)dion in 40 ml Tetrahydrofuran leitet man unter Rühren und Kühlen mit einem Eisbad Chlorwasserstoffgas ein, wobei sich schon nach kurzer Zeit eine kristalline Suspension bildet, die man noch 15 h bei Raumtemperatur stehen lässt. Nach Filtration des Kristallisats und Waschen des Nutschguts mit Tetrahydrofuran erhält man die Titelverbindung, Smp. 242-243°C.

Beispiel 2: 4-Aminooxy-piperidin-dihydrochlorid

Ein Gemisch von 1,57 g (0,005 Mol) 2-Methoxycarbonyl-(N-(4-piperidyloxy))benzoesäureamid-hydrochlorid und 10 ml 2N Salzsäure wird 2 h bei 100° erhitzt und analog Beispiel 1 aufgearbeitet, wobei man die Titelverbindung erhält, Smp. 182°C (Zers.).

Die Ausgangsverbindung wird wie folgt hergestellt:

a) 2-Methoxycarbonyl-(N-(4-piperidyloxy))benzoesäureamid-hydrochlorid

Ein Gemisch von 5 g (0,01443 Mol) 2-(N-BOC-4-piperidyloxy)-1H-isoindol-1,3(2H)-dion und 100 ml einer 1,3 N Lösung von Chlorwasserstoff in Methanol wird 2 h bei Raumtemperatur gerührt und dann im Vakuum eingedampft. Bei der fraktionierten Umkristallisation des Rückstands aus Ethanol fällt zuerst 2-(4-Piperidyloxy)-1H-isoindol1,3(2H)-dion-hydrochlorid, Smp. 240-241°C aus, und beim anschliessenden Einengen der Mutterlauge die Titelverbindung, Smp. 179-180°C (Zers.).

Beispiel 3: 3-Aminooxymethyl-piperidin-dihydrochlorid

Ausgehend von 6 g (0,0166 Mol) 2-(N-BOC-3-piperidylmethoxy)-1H-isoindol- 1,3(2H)-dion und 40 ml 6 N Salzsäure erhält man analog Beispiel 1 die Titelverbindung mit einem Wassergehalt von 1,74 %, Smp. 191°C (Zers.).

Die Ausgangsverbindung wird wie folgt hergestellt:

a) 2-(N-BOC-3-piperidylmethoxy)-1H-isoindol-1,3(2H)-dion

Analog Beispiel 1a werden 6,46 g (0,03 Mol) N-BOC-3-hydroxymethyl-piperidin (vgl. EP 0 279 681), 4,90 g (0,03 Mol) N-Hydroxyphthalimid, 7,87 g (0,03 Mol) Triphenylphosphin und 5,3 ml (0,0317 Mol) Azodicarbonsäure-diethylester (93 %) in 70 ml Tetrahydrofuran umgesetzt. Nach Reinigung des Rohprodukts mittels Flashchromatographie, an Kieselgel unter Verwendung von Essigester-Hexan-Gemischen (1:3 bzw. 1:2) und Eindampfen der produkthaltigen Fraktionen erhält man die Titelverbindung als kristallinen Rückstand, Smp. 92-94°C.

Beispiel 4: 2-(2-Aminooxyethyl)-piperidin-dihydrochlorid

Ein Gemisch von 6 g (0,01602 Mol) 2-(N-BOC-2-piperidylethoxy)-1H-isoindol-1,3 (2H)dion und 40 ml 6 N Salzsäure wird analog Beispiel 1 umgesetzt. Nach zweimaliger Kristallisation aus Ethanol/Ether erhält man die Titelverbindung, Smp. 129-132°C (Zers.).

Die Ausgangsverbindung wird wie folgt hergestellt:

a) 2-(N-BOC-2-piperidylethoxy)-1H-isoindol-1,3(2H)-dion

Analog Beispiel 1a werden 6,9 g (0,03 Mol) N-BOC-2-(2-hydroxyethyl)-piperidin (vgl. EP 0 289 842), 4,9 g (0,03 Mol) N-Hydroxyphthalimid, 7,87 g (0,03 Mol) Triphenylphosphin und 5,3 ml (0,0317 Mol) Azodicarbonsäure-diethylester (93 %) in 70 ml Tetrahydrofuran umgesetzt. Nach Abtrennen der Nebenprodukte (1,2-Hydrazindicarbonsäurediethylester und Triphenylphosphinoxid) und Einengen der etherischen Filtrate erhält man die Titelverbindung als kristallinen Rückstand, Smp. 108°C.

Beispiel 5: 4-Aminooxymethyl-piperidin-dihydrochlorid

Ein Gemisch von 0,5 g (0,00217 Mol) N-BOC-4-aminooxymethyl-piperidin und 10 ml 1,3 N methanolischer Salzsäure wird 1 h bei Raumtemperatur gerührt und anschliessend im Vakuum eingedampft. Nach Umkristallisation des Rückstands aus Methanol/Ether erhält man die Titelverbindung, Smp. 199-200°C (Zers.).

Die Ausgangsverbindungen werden wie folgt hergestellt:

a) N-BOC-4-aminooxymethyl-piperidin

Ein Gemisch von 3,5 g (0,00971 Mol) 2-(N-BOC-4-piperidylmethoxy)-1H-isoindol1,3(2H)-dion und 20 ml Hydrazinhydrat wird 2 h bei Raumtemperatur gerührt, anschliessend mit 75 ml Ether versetzt und noch 2 h weitergerührt. Man trennt dann die organische Phase ab und extrahiert die Hydrazinhydratphase zweimal mit je 50 ml Ether. Die vereinigten etherischen Phasen werden mit Wasser und gesättigter Natriumchloridlösung gewaschen, über Natriumsulfat getrocknet und im Vakuum eingedampft. Man erhält die Titelverbindung in Form eines farblosen Öls, $R_f$: 0,60 [DC-Fertigplatten Kieselgel 60 $F_{254}$;Fliessmittel: Methylenchlorid-Methanol (9:1)].

b) 2-(N-BOC-4-piperidylmethoxy)-1H-isoindol-1,3(2H)-dion

Analog Beispiel 1a, jedoch unter Einhaltung einer Reaktionsdauer von 17 h, erhält man ausgehend von 6,46 g (0,03 Mol) N-BOC-4-hydroxymethyl-piperidin (vgl. EP 0 317 997), 4,9 g (0,03 Mol) N-Hydroxyphthalimid, 7,87 g (0,03 Mol) Triphenylphosphin, 5,3 ml (0,0317 Mol) Azodicarbonsäure-diethylester (93 %) und 120 ml Tetrahydrofuran die Titelverbindung, Smp. 109- 110°C.

Beispiel 6: 2-Aminooxymethyl-piperazin-trihydrochlorid

Ein Gemisch von 2 g (0,00433 Mol) 2-(1,4-Di-BOC-2-piperazinylmethoxy)-1H-isoindol1,3(2H)-dion und 20 ml

6 N Salzsäure wird 2,5 h unter Rückfluss erhitzt und analog Beispiel 1 aufgearbeitet. Nach zweimaliger Umkristallisation aus Methanol/Wasser/Ether erhält man die Titelverbindung mit einem Wassergehalt von 5,82 %, Smp. 130-135°C (Zers.).

Die Ausgangsverbindungen werden wie folgt hergestellt:

a) 2-(1,4-Di-BOC-2-piperazinylmethoxy)-1H-isoindol-1,3(2H)-dion

Analog Beispiel 4b erhält man ausgehend von 15,82 g (0,05 Mol) 1,4-Di-BOC-2hydroxymethyl-piperazin, 8,16 g (0,05 Mol) N-Hydroxyphthalimid, 13,11 g (0,05 Mol) Triphenylphosphin, 8,85 ml (0,0529 Mol) 1,2-Azodicarbonsäure-diethylester (93 %) und 100 ml Tetrahydrofuran die Titelverbindung, Smp. 154-155°C.

b) 1,4-Di-BOC-2-hydroxymethyl-piperazin

Ein Gemisch von 8,71 g (0,075 Mol) 2-Hydroxymethyl-piperazin [vgl. J. Med. Chem. 33, 142-146(1990)], 100 ml Acetonitril und 50 ml Wasser wird bei 5-10°C unter Rühren tropfenweise mit einer Lösung von 34,4 g (0,1576 Mol) Di-tert.-butyl-dicarbonat in 100 ml Acetonitril versetzt. Man rührt das Reaktionsgemisch noch 15 h bei Raumtemperatur und dampft es dann im Vakuum ein. Nach Kristallisation des Rückstands aus Essigester/Hexan erhält man die Titelverbindung, Smp. 120-121°C.

Beispiel 7: 2-Aminooxymethyl-morpholin-dihydrochlorid

Eine Lösung von 0.9 g (2.7 mMol) N,N′-Di-BOC-2-aminooxymethyl-morpholin in 10 ml Ethanol wird mit 10 ml einer 2,2N ethanolischen Salzsäure versetzt und 16 h bei Raumtemperatur gerührt. Die auskristallisierte Titelverbindung wird abgesaugt, mit wenig Ethanol und Ether gewaschen und getrocknet, Smp. 185°C (mit Zers.).

Die Ausgangsverbindungen werden wie folgt hergestellt:

a) N-2-Hydroxyethyl-3-(1-ethoxyethylidenaminooxy)-2-hydroxy-propylamin

Eine Lösung von 6,4 g (49.6 mMol) 0-2,3-Epoxypropyl-acethydroxamsäure-ethylester in 80 ml Isopropanol wird mit 8 ml (131 mMol) Ethanolamin versetzt und 16 h bei 60° C gerührt. Das Reaktionsgemisch wird eingedampft und der Rückstand durch Chromatographie an 250 g Kieselgel mit einem Gemisch von Methylenchlorid:Methanol 10:1 und danach mit Methylenchlorid:Methanol:konz. Ammoniak 300:50:1 gereinigt. Man erhält so das Ausgangsprodukt a als gelbes Oel, $R_f$-Wert = 0.12 (Kieselgel/Methylenchlorid:Methanol:konz. Ammoniak 300:50:1).

b) N,N′-BOC-N-2-hydroxyethyl-3-aminooxy-2-hydroxy-propylamin

Eine Lösung von 5,2 g (23,6 mMol) des Ausgangsprodukts a in 100 ml 2N Salzsäure wird 4 h am Rückfluss gekocht. Nach dem Abkühlen werden unter Rühren nacheinander 100 ml 2N Natronlauge, 200 ml Tetrahydrofuran, 2 g Natriumcarbonat und eine Lösung von 11 g Di-tert.-butyldicarbonat in 100 ml Tetrahydrofuran zugegeben und man lässt 24 h bei Raumtemperatur nachreagieren. Das zweiphasige Reaktionsgemisch wird nun mit 500 ml Ether verdünnt. Die organische Phase wird abgetrennt, mit Wasser gewaschen, über Magnesiumsulfat getrocknet, filtriert und eingedampft. Das zurückgebliebene Oel wird mit einem Gemisch von Hexan:Essigester 1:1 über 250 g Kieselgel chromatographiert und liefert die Ausgangsverbindung b als gelbes Oel, $R_f$-Wert = 0,06 (Kieselgel/Hexan:Essigester 1:2).

c) N,N′-Di-BOC-2-aminooxymethyl-morpholin

Eine Lösung von 1,2 g (3,4 mMol) des Ausgangsprodukts b in 10 ml Pyridin wird unter Rühren und Feuchtigkeitsausschluss bei 0°C mit einer Lösung von 0,67 g (3,5 mMol) Tosylchlorid in 10 ml Methylenchlorid tropfenweise versetzt. Man rührt 1 h im Eisbad und 16 h bei Raumtemperatur, bevor man 1 ml Wasser zugibt und das Reaktionsgemisch zur Trockne eindampft. Der Rückstand wird zwischen 100 ml Ether und 50 ml Wasser verteilt. Die organische Phase wird abgetrennt und mit Wasser, einer gesättigten Natriumhydrogencarbonatlösung und wieder Wasser gewaschen, über Magnesiumsulfat getrocknet, filtriert und eingedampft. Der Rückstand wird mit einem Gemisch von Hexan: Essigester 2:1 über 400 g Kieselgel chromatographiert und liefert das Ausgangsprodukt c als weisse Kristalle, $R_f$-Wert 0,16 (Kieselgel/Hexan:Essigester 2:1), Smp. 106- 108°C. $^1$H-NMR ($CDCl_3$): δ 1,46 (s, 18H); 2,62-2,81 (t, 1H); 2,82-3,05 (t, 1H); 3,46-3,61 (m, 1H); 3,64-3,77 (m, 1H); 3,80-4,00 (m, 5H); 7,3 (s, 1H).

d) N,N′-Di-BOC-2-aminooxymethyl-morpholin kann auch wie folgt hergestellt werden: 2,2 g (14 mMol) N-2-Hydroxyethyl-3-aminooxy-2-hydroxy-propylamin-dihydrochlorid werden in einem Teflonreaktor bei -78°C in 25 ml trockenem Fluorwasserstoff gelöst. In diese Lösung werden 5,4 g (50 mMol) Schwefeltetrafluorid eingeleitet, der Reaktor wird verschlossen und 24 h bei 0°C gerührt. Danach wird der Reaktor entgast und der Rückstand in 20 ml 2N HCl gelöst. Diese Lösung wird filtriert und nacheinander mit 100 ml Tetrahydrofuran verdünnt, mit festem Natriumhydrogencarbonat neutralisiert und mit einer Lösung von 7,6 g Di-tert.-butyldicarbonat in 100 ml Tetrahydrofuran tropfenweise versetzt. Das Reaktionsgemisch wird 16 h bei Raumtemperatur gerührt und mit 100 ml Ether verdünnt. Die organische Phase wird abgetrennt, mit Wasser, einer gesättigten Natriumhydrogencarbonatlösung und wieder Wasser gewaschen, über Magnesiumsulfat getrocknet, filtriert und eingedampft. Der Rückstand wird mit einem Gemisch

von Hexan:Essigester 2:1 über 400 g Kieselgel chromatographiert und liefert das Ausgangsprodukt c als weisse Kristalle, $R_f$-Wert 0,16 (Kieselgel/Hexan:Essigester 2:1), Smp. 106-108°C. 1H-NMR ($CDCl_3$): δ 1,46 (s, 18H); 2,62-2,81 (t, 1H); 2,82-3,05 (t, 1H); 3,46-3,61 (m, 1H); 3,64-3,77 (m, 1H); 3,80-4,00 (m, 5H); 7,3 (s, 1H).

Beispiel 8: 2-Aminooxymethyl-morpholin-dihydrochlorid

3,2 g (14 mMol) N-2-Hydroxyethyl-3-aminooxy-2-hydroxy-propylamin-dihydrochlorid werden in einem Teflonreaktor bei -78°C in 25 ml trockenem Fluorwasserstoff gelöst. In diese Lösung werden 5,4 g (50 mMol) Schwefeltetrafluorid eingeleitet, der Reaktor wird verschlossen und 24 h bei 0°C gerührt. Danach wird der Reaktor entgast und der Rückstand in 20 ml 2N Salzsäure gelöst. Diese Lösung wird filtriert und zur Trockne eingedampft. Der ölige Rückstand wird in wenig Wasser aufgenommen und über 200 ml Dowex 1 ($Cl^-$-Form) chromatographiert. Die die Titelverbindung enthaltenden Fraktionen werden vereinigt und eingedampft. Der Rückstand wird aus Ethanol und Ether kristallisiert, Smp. 185°C.

Beispiel 9: 2-Aminooxymethyl-morpholin-dihydrochlorid

Eine Lösung von 2,5 g (20 mMol) Acethydroxamsäure-ethylesternatriumsalz und 10,8 g (21 mMol) 4-Triphenylmethyl-2-(p-toluolsulfonyloxymethyl)morpholin [Chem. Pharm. Bull. 33, 3766 (1985)] in 50 ml N,N-Dimethylformamid wird 16 h bei 100°C gerührt. Nach dem Abkühlen wird das Reaktionsgemisch auf 400 ml Wasser gegossen. Das ausgefallene Produkt wird abgesaugt, mit Wasser gewaschen und in 100 ml Ethanol aufgenommen. Diese Lösung wird mit 100 ml 2N Salzsäure versetzt, 4 h am Rücfluss gekocht abgekühlt, filtriert und das Filtrat eingedampft. Der Rückstand wird aus Ethanol und Ether kristallisiert und stellt die Titelverbindung dar, Smp. 185°C.

Beispiel 10: 2-Aminooxymethyl-piperazin-trihydrochlorid

Ein Gemisch von 0,45 g ( 1,35 mMol) 2-(N-BOC-Aminooxymethyl)-4-N-BOC-piperazin in 10 ml Ethanol wird mit 10 ml 2,2 N ethanolischer Salzsäure versetzt, 16 h bei Raumtemperatur gerührt und anschliessend im Vakuum eingedampft. Nach Umkristallisation des Rückstands aus Methanol/Wasser/Ether erhält man die Titelverbindung, Smp. 130- 135°C (Zers.).

Die Ausgangsverbindungen werden wie folgt hergestellt:

a) N,N′-Di-BOC-N-2-tosyloxyethyl-3-aminooxy-2-tosyloxy-propylamin

Eine Lösung von 1,2 g (3,4 mMol) N,N′-Di-BOC-N-2-hydroxyethyl-3-aminooxy-2-hydroxy-propylamin in 10 ml Pyridin wird unter Rühren und Feuchtigkeitsausschluss bei 0°C mit einer Lösung von 1,34 g (7,0 mMol) Tosylchlorid in 10 ml Methylenchlorid tropfenweise versetzt. Man rührt 16 h im Eisbad, giesst auf Eiswasser und extrahiert mit Ether. Die Etherphase wird mit Wasser, verdünnter eiskalter Salzsäure, einer gesättigten Natriumhydrogencarbonatlösung und wieder Wasser gewaschen, über Magnesiumsulfat getrocknet, filtriert und eingedampft, wobei man die Ausgangsverbindung a erhält.

b) 2-(N-BOC-Aminooxymethyl)-4-N-BOC-piperazin

Ein Gemisch von N,N′-Di-BOC-N-2-tosyloxyethyl-3-aminooxy-2-tosyloxy-propylamin in einer 12%igen ethanolischen Ammoniaklösung wird im Autoklav 5 h auf 100°C erwärmt. Danach wird das Reaktionsgemisch filtriert, eingedampft und der Rückstand in Ether aufgenommen. Die Etherlösung wird mit verdünnter Natronlauge und Wasser gewaschen, über Natriumsulfat getrocknet und im Vakuum eingedampft, wobei man die Ausgangsverbindung b erhält (vgl. Archiv der Pharmazie 291, 3 (1958)).

Beispiel 11: 2-Aminooxymethyl-thiomorpholin-dihydrochlorid

Analog Beispiel 10 wird 2-(N-BOC-Aminooxymethyl)-4-N-BOC-thiomorpholin mit ethanolischer Salzsäure behandelt, wobei man die Titelverbindung erhält.

Die Ausgangsverbindung wird wie folgt hergestellt:

a) 2-(N-BOC-Aminooxymethyl)-4-N-BOC-thiomorpholin

Ein Gemisch von N,N′-Di-BOC-N-2-tosyloxyethyl-3-aminooxy-2-tosyloxy-propylamin und Natriumsulfid-Hydrat in Ethanol wird 4 h am Rückfluss gekocht. Das Reaktionsgemisch wird filtriert, eingedampft und der Rückstand in Ether aufgenommen. Die Etherlösung wird mit Wasser gewaschen, über Natriumsulfat getrocknet und im Vakuum eingedampft, wobei man die Ausgangsverbindung a erhält (vgl. Austral. J. Chem. 9, 397 (1956)).

Beispiel 12: 4-Aminooxy-piperidin-dihydrochlorid

Ein Gemisch von 1,08 g (0,005 Mol) N-BOC-4-aminooxy-piperidin und 25 ml 1,3 N methanolischer Salzsäure wird 8 h bei Raumtemperatur gerührt und anschliessend im Vakuum eingedampft. Nach Umkristallisation des Rückstandes aus Methanol/Ether erhält man die Titelverbindung, Smp. 182 °C (Zers.).

Die Ausgangsverbindung wird wie folgt hergestellt:

a) N-BOC-4-aminooxy-piperidin

Ein Gemisch von 3 g (0,00866 Mol) 2-(N-BOC-4-piperidyloxy)-1H-isoindol- 1,3(2H)-dion (Beispiel 1 a)) und 17 ml Hydrazinhydrat wird 1 h bei Raumtemperatur gerührt. Anschliessend versetzt man das Reaktionsgemisch mit 15 ml Wasser und 70 ml Ether und rührt noch 1,5 h weiter. Nach Aufarbeiten analog Beispiel 5 a) erhält man die Titelverbindung als kristallinen Rückstand, Smp. 51 - 52 °C.

Beispiel 13: 4-Aminooxy-piperidin-sulfat

Eine Lösung von 2,32 g (0,02 Mol) 4-Aminooxypiperidin in 50 ml Ethanol wird bei Raumtemperatur unter Rühren mit 20 ml 2 N Schwefelsäure (0,02 Mol) versetzt und das Reaktionsgemisch anschliessend auf 5 °C abgekühlt. Nach Filtration und Waschen des Niederschlages mit Ethanol und Ether erhält man die Titelverbindung, Smp. 245 - 247 °C (Zers.).

Die Ausgangsverbindung wird wie folgt hergestellt:

a) 4-Aminooxy-piperidin

Eine Lösung von 16 g (0,0846 Mol) 4-Aminooxy-piperidin-dihydrochlorid (Beispiel 1, 2, 12) in 10 ml Wasser wird auf eine mit Amberlite® IRA-400 (Anionenaustauscher in der Hydroxid-Form) beschickte Säule aufgetragen und mit Wasser eluiert. Nach Eindampfen der produkthaltigen Fraktionen im Vakuum bei 40 °C erhält man die Titelverbindung in Form eines farblosen Öls, $R_f$: 0,11 (Kieselgel-Dünnschichtchromatographie, Laufmittel Methylenchlorid/Methanol/konz. Ammoniak 40/10/1 (v/v));

Beispiel 14: 4-Aminooxy-piperidin-benzolsulfonat

Zu einer Lösung von 2,32 g (0,02 Mol) 4-Aminooxy-piperidin in 20 ml Ethanol gibt man eine Lösung von 3,16 g (0,02 Mol) Benzolsulfonsäure in 30 ml Ethanol. Nach Einenben auf ein Volumen von ca. 25 ml tropft man unter Rühren 30 ml Ether zu. Es bildet sich ein kristalliner Niederschlag, der abfiltriert und mit Ether gewaschen wird. Die so erhaltene Titelverbindung schmilzt bei 110 - 112 °C.

Beispiel 15: 4-Aminooxy-piperidin-adipat

Zu einer 35 °C warmen Lösung von 2,32 g (0,02 Mol) 4-Aminooxy-piperidin und 2,92 g (0,02 Mol) Adipinsäure in 70 ml Ethanol gibt man unter Rühren 50 ml Ether. Nach Abkühlen auf Raumtemperatur, Filtration des gebildeten kristallinen Niederschlabs und Waschen desselben mit Ether erhält man die Titelverbindung, Smp. 97 - 98 °C.

Beispiel 16:

Kapseln enthaltend 0,25 g Wirkstoff, z.B. eine der Verbindungen der Beispiele 1-15, können wie folgt hergestellt werden:

Zusammensetzung (für 5000 Kapseln)

| | |
|---|---|
| Wirkstoff | 1250 g |
| Talk | 180 g |
| Weizenstärke | 120 g |
| Magnesiumstearat | 80 g |
| Laktose | 20 g |

Die pulverförmigen Substanzen werden durch ein Sieb mit einer Maschenweite von 0,6 mm getrieben und gemischt. Portionen von je 0,33 g des Gemisches werden mittels einer Kapselfüllmaschine in Gelatine-Kapseln abgefüllt.

## Patentansprüche

1. Verbindungen der Formel

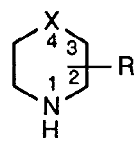

(I),

worin X für Methylen, Imino, Sauerstoff oder Schwefel steht und R einen Rest

$$-(CH_2)_n-O-NH_2$$

bezeichnet, worin n 0, 1 oder 2 bedeutet, mit der Massgabe, dass n von 0 verschieden ist, wenn R in Position 2 gebunden ist, und Salze davon.

2. Verbindungen der Formel I gemäss Anspruch 1, in denen X für Methylen, Imino oder Sauerstoff steht, R in Position 2 oder 3 gebunden ist und n 1 oder 2 ist, und Salze davon.

3. Verbindungen der Formel I gemäss Anspruch 1, in denen X für Methylen oder Sauerstoff steht, R in Position 2 oder 3 gebunden ist und n 1 oder 2 ist, und Salze davon.

4. Verbindungen der Formel I gemäss Anspruch 1, in denen X für Methylen oder Sauerstoff steht, R in Position 3 gebunden ist und n 1 ist, und Salze davon.

5. Verbindungen der Formel I gemäss Anspruch 1, in denen X Methylen bedeutet, R in Position 4 gebunden ist, und n 0 oder 1 ist, und Salze davon.

6. Pharmazeutisch annehmbare Salze von Verbindungen der Formel I gemäss einem der Ansprüche 1 bis 5.

7. Die Verbindung der Formel I gemäss Anspruch 1, worin X für Methylen steht und im Rest R n gleich 0 ist, wobei R in 4-Stellung des Ringes steht, mit dem Namen 4-Aminooxy-piperidin, und pharmazeutisch annehmbare Salze davon.

8. Die Verbindung der Formel I gemäss Anspruch 1, worin X für Sauerstoff steht und im Rest R n gleich 1 ist, wobei R in 3-Stellung des Ringes steht, mit dem Namen 2-Aminooxymethyl-morpholin, und pharmazeutisch annehmbare Salze davon.

9. Die Verbindung der Formel I gemäss Anspruch 1, worin X für Methylen steht und im Rest R n gleich 1 ist, wobei R in 3-Stellung des Ringes mit der Numerierung gemäss Formel I steht, mit dem Nammen 3-Aminooxymethyl-piperidin, und pharmazeutisch annehmbare Salze davon.

10. Eine Verbindung der Formel I gemäss einem der Ansprüche 1 bis 5 und 7 bis 9, oder ein pharmazeutisch annehmbares Salz davon, zur Anwendung in einem Verfahren zur therapeutischen Behandlung des tierischen oder menschlichen Körpers.

11. Verwendung einer Verbindung der Formel Igemäss einem der Ansprüche 1 bis 5 und 7 bis 9, oder eines pharmazeutisch annehmbaren Salzes davon, zur Herstellung von pharmazeutischen Präparaten zur Anwendung für die Behandlung von Erkrankungen, die auf eine Hemmung der Ornithindecarboxylase ansprechen.

12. Verfahren zur Herstellung von Verbindungen der Formel I oder deren Salzen gemäss Anspruch 1, dadurch gekennzeichnet, dass man
(a) aus einer Verbindung der Formel I, worin mindestens eine Aminogruppe geschützt ist, d. h. einer Verbindung der Formel

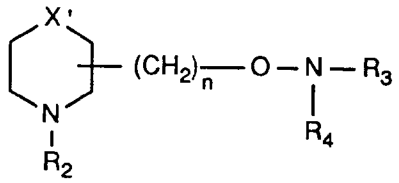

(II)

oder einem Salz davon, worin X′ für Methylen, Sauerstoff, Schwefel oder N-$R_1$ steht, n 0, 1 oder 2 bedeutet, mit der Massgabe, dass n von 0 verschieden ist, wenn der Substituent -$(CH_2)_n$-O-$NR_3R_4$ in Position 2 gebunden ist, und $R_1$, $R_2$, $R_3$ und $R_4$ unabhängig voneinander für eine Aminoschutzgruppe oder Wasserstoff stehen, mit der Massgabe, dass mindestens eine der Gruppen $R_1$, $R_2$, $R_3$ und $R_4$ eine Aminoschutzgruppe bedeutet, oder worin $R_1$ und $R_2$ unabhängig voneinander für eine Aminoschutzgruppe oder Wasserstoff stehen und $R_3$ zusammen mit $R_4$ eine bivalente Aminoschutzgruppe bildet, die Aminoschutzgruppe(n) abspaltet, oder

(b) zur Herstellung von Verbindungen der Formel I oder eines Salzes davon, worin X Imino oder Methylen bedeutet, eine Verbindung der Formel

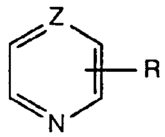

(III)

oder ein Salz davon, worin Z für Stickstoff oder die Methingruppe CH steht, und R die unter Formel I angegebene Bedeutung hat, reduziert, oder

(c) zur Herstellung von Verbindungen der Formel I oder eines Salzes davon, worin X Imino, Sauerstoff oder Schwefel bedeutet, R in Position 2 oder 3 gebunden ist, und n 1 oder 2 ist, eine Verbindung der Formel

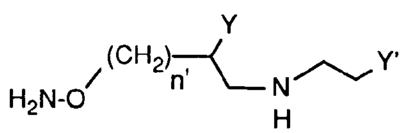

(IV),

worin n′ für 1 oder 2 steht und einer der Reste Y und Y′ Hydroxyl, Amino oder Sulfhydryl darstellt, und der andere eine nucleofuge Abgangsgruppe bedeutet, oder ein Salz davon cyclisiert, oder

(d) eine Verbindung der Formel

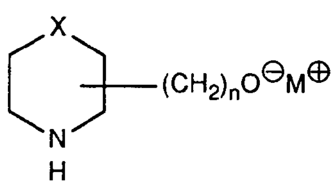

(V)

oder ein Salz davon, worin X und n die unter Formel I angegebenen Bedeutungen haben, mit der Massgabe, dass n von 0 verschieden ist, wenn der Substituent -$(CH_2)_nO^{\ominus}M^{\oplus}$ in Position 2 gebunden ist, und $M^{\oplus}$ für ein Metallkation steht, mit einer Verbindung der Formel $H_2N$-A′ oder einem Salz davon, worin A′ eine nucleofuge Abgangsgruppe ist, um setzt, und

gewünschtenfalls eine erhältliche Verbindung der Formel I in eine andere Verbindung der Formel I überführt, ein erhältliches Enantiomerengemisch in die Enantiomeren aufspaltet und/oder eine erhältliche freie Verbindung der Formel I in ein Salz überführt oder ein erhältliches Salz in die freie Verbindung der Formel I oder in ein anderes Salz umwandelt.

**Patentansprüche für folgende Vertragsstaaten : ES, GR**

1. Verfahren zur Herstellung von Verbindungen der Formel I

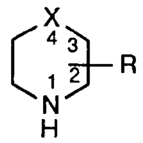

(I),

worin X für Methylen, Imino, Sauerstoff oder Schwefel steht und R einen Rest

$$-(CH_2)_n-O-NH_2$$

bezeichnet, worin n 0, 1 oder 2 bedeutet, mit der Massgabe, dass n von 0 verschieden ist, wenn R in Position 2 gebunden ist, und von deren Salzen, dadurch gekennzeichnet, dass man

(a) aus einer Verbindung der Formel I, worin mindestens eine Aminogruppe geschützt ist, d. h. einer Verbindung der Formel

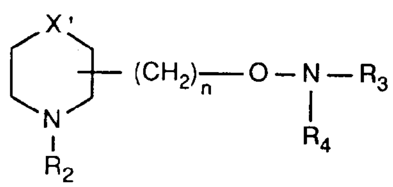

(II)

oder einem Salz davon, worin X′ für Methylen, Sauerstoff, Schwefel oder N-$R_1$ steht, n 0, 1 oder 2 bedeutet, mit der Massgabe, dass n von 0 verschieden ist, wenn der Substituent $-(CH_2)_n-O-NR_3R_4$ in Position 2 gebunden ist, und $R_1$, $R_2$, $R_3$ und $R_4$ unabhängig voneinander für eine Aminoschutzgruppe oder Wasserstoff stehen, mit der Massgabe, dass mindestens eine der Gruppen $R_1$, $R_2$, $R_3$ und $R_4$ eine Aminoschutzgruppe bedeutet, oder worin $R_1$ und $R_2$ unabhängig voneinander für eine Aminoschutzgruppe oder Wasserstoff stehen und $R_3$ zusammen mit $R_4$ eine bivalente Aminoschutzgruppe bildet, die Aminoschutzgruppe(n) abspaltet, oder

(b) zur Herstellung von Verbindungen der Formel I oder eines Salzes davon, worin X Imino oder Methylen bedeutet, eine Verbindung der Formel

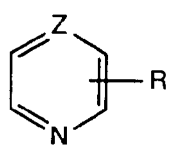

(III)

oder ein Salz davon, worin Z für Stickstoff oder die Methingruppe CH steht, und R die unter Formel I angegebene Bedeutung hat, reduziert, oder

(c) zur Herstellung von Verbindungen der Formel I oder eines Salzes davon, worin X Imino, Sauerstoff oder Schwefel bedeutet, R in Position 2 oder 3 gebunden ist, und n 1 oder 2 ist, eine Verbindung der Formel

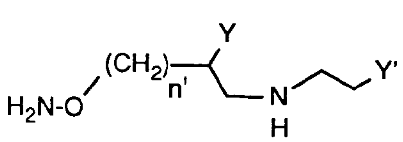

(IV),

worin n′ für 1 oder 2 steht und einer der Reste Y und Y′ Hydroxyl, Amino oder Sulfhydryl darstellt, und der andere eine nucleofuge Abgangsgruppe bedeutet, oder ein Salz davon cyclisiert, oder

(d) eine Verbindung der Formel

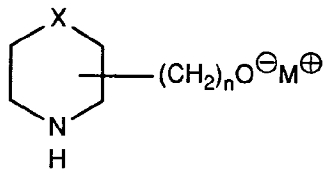

(V)

oder ein Salz davon, worin X und n die unter Formel I angegebenen Bedeutungen haben, mit der Massgabe, dass n von 0 verschieden ist, wenn der Substituent $-(CH_2)_nO^{\ominus}M^{\oplus}$ in Position 2 gebunden ist, und $M^{\oplus}$ für ein Metallkation steht, mit einer Verbindung der Formel $H_2N$-A' oder einem Salz davon, worin A' eine nucleofuge Abgangsgruppe ist, umsetzt, und
gewünschtenfalls eine erhältliche Verbindung der Formel I in eine andere Verbindung der Formel I überführt, ein erhältliches Enantiomerengemisch in die Enantiomeren aufspaltet und/oder eine erhältliche freie Verbindung der Formel I in ein Salz überführt oder ein erhältliches Salz in die freie Verbindung der Formel I oder in ein anderes Salz umwandelt.

**2.** Verfahren gemäss Anspruch 1 zur Herstellung einer Verbindung der Formel I, in der X für Methylen, Imino oder Sauerstoff steht, R in Position 2 oder 3 gebunden ist und n 1 oder 2 ist, und von deren Salzen, in dem man die entsprechend substituierten Ausgangsverbindungen verwendet.

**3.** Verfahren gemäss Anspruch 1 zur Herstellung einer Verbindung der Formel I, in der X für Methylen oder Sauerstoff steht, R in Position 2 oder 3 gebunden ist und n 1 oder 2 ist, und von deren Salzen, in dem man die entsprechend substituierten Ausgangsverbindungen verwendet.

**4.** Verfahren gemäss Anspruch 1 zur Herstellung einer Verbindung der Formel I, in der X für Methylen oder Sauerstoff steht, R in Position 3 gebunden ist und n 1 ist, und von deren Salzen, in dem man die entsprechend substituierten Ausgangsverbindungen verwendet.

**5.** Verfahren gemäss Anspruch 1 zur Herstellung einer Verbindung der Formel I, in der X für Methylen steht, R in Position 4 gebunden ist, und n 0 oder 1 ist, und von deren Salzen, in dem man die entsprechend subsbtuierten Ausgangsverbindungen verwendet.

**6.** Verfahren gemäss einem der Ansprüche 1 bis 5 zur Herstellung einer Verbindung der Formel I in Form eines pharmazeutsich annehmbaren Salzes, in dem man die entsprechend substituierten Ausgangsverbindungen verwendet.

**7.** Verfahren gemäss Anspruch 1 zur Herstellung einer Verbindung der Formel I, worin X für Methylen steht und im Rest R n gleich 0 ist, wobei R in 4-Stellung des Ringes steht, mit dem Namen 4-Aminooxy-piperidin, und von pharmazeutisch annehmbaren Salzen davon, in dem man die entsprechend substituierten Ausgangsverbindungen verwendet.

**8.** Verfahren gemäss Anspruch 1 zur Herstellung einer Verbindung der Formel I, worin X für Sauerstoff steht und im Rest R n gleich 1 ist, wobei R in 3-Stellung des Ringes steht, mit dem Namen 2-Aminooxymethylmorpholin, und von pharmazeutisch annehmbaren Salzen davon, in dem man die entsprechend substituierten Ausgangsverbindungen verwendet.

**9.** Verfahren gemäss Anspruch 1 zur Herstellung einer Verbindung der Formel I, worin X für Methylen steht und im Rest R n gleich 1 ist, wobei R in 3-Stellung des Ringes mit der Numerierung gemäss Formel I steht, mit dem Namen 3-Aminooxymethyl-piperidin, und von pharmazeutisch annehmbaren Salzen davon, in dem man die entsprechend substituierten Ausgangsverbindungen verwendet.

**10.** Verfahren zur Herstellung eines pharmazeutischen Präparates, dadurch gekennzeichnet, dass man eine gemäss einem Verfahren eines der Ansprüche 1 bis 5 und 7 bis 9 erhaltene Verbindung der Formel I, oder ein pharmazeutisch verwendbares Salz davon, mit mindestens einem pharmazeutisch verwendbaren Trägermaterial versetzt.

**11.** Verfahren zur Herstellung eines pharmazeutischen Präparates, dadurch gekennzeichnet, dass man eine

gemäss einem Verfahren eines der Ansprüche 1 bis 5 und 7 bis 9 erhaltene Verbindung der Formel I, oder ein pharmazeutisch verwendbares Salz davon, mit mindestens einem pharmazeutisch verwendbaren Trägermaterial versetzt, wobei der Wirkstoff im erhaltenen Präparat in einem Gewichtsanteil von 5 bis 95% vorliegt.